(19)

Europäisches
Patentamt
European
Patent Office
Office européen
des brevets

(11) **EP 4 160 617 A1**

(12) **EUROPEAN PATENT APPLICATION**

(43) Date of publication:
**05.04.2023 Bulletin 2023/14**

(21) Application number: **21306385.2**

(22) Date of filing: **04.10.2021**

(51) International Patent Classification (IPC):
***G16H 50/30*** (2018.01)

(52) Cooperative Patent Classification (CPC):
**G16H 50/30; G16H 20/00; G16H 40/63;**
**G16H 50/20; G16H 50/70**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(71) Applicant: **Implicity**
**75001 Paris (FR)**

(72) Inventors:
• **GIRAULT, Emmanuel**
**75001 Paris (FR)**

• **VINCENT, Louis**
**75001 Paris (FR)**
• **GENTILS, Marika**
**75001 Paris (FR)**
• **IBNOUHSEIN, Issam**
**75001 Paris (FR)**
• **PERLMUTTER, David**
**75001 Paris (FR)**
• **BONNET, Jean-Luc**
**75001 Paris (FR)**
• **ROSIER, Arnaud**
**75001 Paris (FR)**

(74) Representative: **Icosa**
**83 avenue Denfert-Rochereau**
**75014 Paris (FR)**

(54) **METHOD FOR PREDICTING AN EVOLUTION OF A PATIENT'S HEART- RELATED CONDITION**

(57)     The invention relates to a computer-implemented method for predicting an evolution of at least one heart-related condition of a patient, the method including, for each heart-related condition:

a) based on a set of inputs, at least part of the set of inputs being representative of a temporal evolution of a corresponding health variable among a predetermined set of health variables of the patient, computing a set of transformed variables, the set of transformed variables being a representation of the set of inputs in a predetermined latent data representation space;

b) determining, based on the computed set of transformed variables, at least one index value, each representative of a respective risk of an unfavorable outcome of the heart-related condition.

**FIG. 1**

**Description**

**FIELD OF INVENTION**

The present invention relates to a computer-implemented method for predicting an evolution of at least one heart-related condition of a patient.

The invention also relates to a computer product program and to a prediction system for carrying out said method.

The invention relates to the field of computer science, and more specifically to the monitoring and the prediction of a health status of a patient.

**BACKGROUND OF INVENTION**

Cardiovascular conditions cover a large spectrum of diseases. The most common are: heart failure, ischemia and myocardial infarction, hypertension, coronary artery diseases, arrhythmias (bradycardia and tachycardia), valvular heart diseases, etc. Some non-cardiovascular diseases are associated to a cardiovascular disease (CVD), such as atrial fibrillation which is highly correlated to the risk of stroke. Another example is sleep apnea which can lead to ventricular arrhythmias. CVDs are common, have poor survival rates, and are increasing worldwide. More than 30% of overall causes of death are related to a CVD. Hence, it is estimated that over 23 million people will die each year due to a CVD by 2030. It is established that up to 90% of these conditions are preventable if the risk factors are identified. These factors are of several types: medical, social, behavioral or genetic.

Heart failure, which is part of the aforementioned cardiovascular conditions, occurs when the heart fails to efficiently pump blood in the vascular system. Such condition may be caused by diseases that lead to a progressive fatigue of the heart, for instance by rendering the heart too weak or too stiff to properly fulfill its function, such as coronary artery disease or high blood pressure.

In developed countries, more than 40 million patients are currently diagnosed with heart failure. Moreover, in developed countries, heart failure represents approximately 1 to 2% of the general population and more than 10% among people over 70 years old. Heart failure is a concerning chronic pathology because it is usually the cause of repeated hospitalizations of a same patient: for instance, patients are readmitted at the hospital several times after their first hospitalization for a cardiac decompensation due to heart failure worsening, and almost half of them die within five years. Moreover, said hospitalizations are costly; indeed, it is estimated that 10% of hospital expenses in France are related to heart failure, that is an annual budget of approximately 1.5 billion euros, and, in the USA, in-patient hospital costs represent more than 10 billion dollars.

Although heart failure is a disease that is difficult to cure, treatments can improve the health status of patients, and therefore extend their life expectancy. Such treatments may also delay the occurrence of heart attacks or other heart-related conditions. However, such treatments need to be provided in a timely manner, *i.e.,* before a worsening of the health status of the patient. Therefore, there is a need to anticipate such worsening.

According to international guidelines, the implantation of a cardiac implantable electronic device (CIED) is recommended in some cases: such as an implantable defibrillator for primary prevention in symptomatic HF patients with left ventricular ejection fraction (LVEF) $\leq$ 35%; and cardiac resynchronization device are restricted to symptomatic patients with LVEF $\leq$ 35%, despite optimal medical therapy, who have left bundle branch block and QRS duration $\geq$ 130 ms.

Nowadays, the prediction of the evolution of heart-related conditions of a patient is performed by the cardiologist, who usually compares a long-term evolution of relevant health variables of the patient to their evolution over a shorter and more recent time interval, and draws conclusions from such comparison.

Moreover, automated method for determining a heart failure risk of a patient are being implemented, such as the method disclosed in document US9622664.

Nevertheless, such known methods are not satisfactory.

Indeed, the human-based method is mainly based on the experience of the healthcare provider, so that, in certain situations, it may be biased. Moreover, the healthcare provider may not be able to efficiently cross-compare a high number of health variables, therefore leading to erroneous predictions.

Moreover, the method of document US9622664 relies on inputs that are not widely available, but are rather specific to a given medical device manufacturer. As a result, prediction of the evolution of a heart-related condition for a patient who is not provided with the appropriate sensors is not possible. Moreover, in such method, cross-comparison of the inputs is insufficient.

A purpose of the invention is therefore to provide a method for predicting the evolution of at least one heart-related condition, that is more objective and reliable than current methods, that does not exclusively rely on the experience of a healthcare provider, and that is not limited to a specific manufacturer device, so that it is applicable to more patients than existing solutions.

## SUMMARY

To this end, the present invention is a method of the aforementioned type, the method including, for each heart-related condition:

a) based on a set of inputs, at least part of the set of inputs being representative of a temporal evolution of a corresponding health variable among a predetermined set of health variables of the patient, computing a set of transformed variables, the set of transformed variables being a representation of the set of inputs in a predetermined latent data representation space;

b) determining, based on the computed set of transformed variables, at least one index value, each representative of a respective risk of an unfavorable outcome of the heart-related condition.

Indeed, thanks to the computation of the set of transformed variables, correlation between the inputs can be found more easily, which renders the determination of each index value easier, while enhancing reliability of such prediction. Moreover, a wide variety of inputs can be taken into account for predicting the evolution of each heart-related condition, which also increases reliability of the prediction, while allowing the method to be performed on inputs that are not necessarily provided by complex or dedicated sensors, so that the prediction method according to the invention is very versatile.

According to other advantageous aspects of the invention, the method includes one or more of the following features, taken alone or in any possible combination:

- step a) includes using a data space transformation model to compute the set of transformed variables, the data space transformation model being trained in order to determine the latent data representation space based on a first training dataset including at least one set of reference inputs, each reference input of a given set of reference inputs being representative of an evolution, over time, of a respective health variable of a respective first reference subject;

- the first training dataset includes at least one healthy set of reference inputs associated to a corresponding healthy patient who has not experienced an unfavorable outcome of the heart-related condition, the data space transformation model being trained so that, in the latent data representation space, each transformed variable of the set of reference transformed variables corresponding to each healthy set of reference inputs has a predefined mean value and is, preferably, normally distributed;

- the loss function, during training of the data space transformation model, includes at least one of the following components:

$$\alpha \left\| f(Y_{i,j}) - \mu \right\|_2^2$$
$$\left\| f(Y_{i,j}) - f(Y'_{i,j}) \right\|$$
$$\frac{\left| f(Y_{i,j}) - f(Y_{i,j+1}) \right|}{\left| Y_{i,j} - Y_{i,j+1} \right|^2}$$

$Y_{i,j}$ being the inputs for patient $i$ at timestamp $t_j$;
$Y'_{i,j}$ being inputs generated by applying a random mask $v_{i,j}$ to $Y_{i,j}$;
$f$ being a transformation function of the data transformation model for representing the set of transformed variables in the latent data representation space;
$\alpha$ being a predetermined hyperparameter;
$\|...\|$ being a predetermined norm; and
$\|...\|_2$ being the Euclidean norm;

- the data space transformation model is an autoencoder including an encoder for receiving the set of inputs, the set of transformed variables being an output of the encoder;

- the loss function, during training of the data space transformation model, includes at least one of the following components:

$$\left\| Y_{i,j} - \left( g \circ f(Y_{i,j}) \right) u_{i,j} \right\|$$
$$\left\| Y'_{i,j} - \left( g \circ f(Y'_{i,j}) \right) v_{i,j} \right\|$$

$Y_{i,j}$ being the inputs for patient $i$ at timestamp $t_j$;

$Y'_{i,j}$ being inputs generated by applying a random mask $v_{i,j}$ to $Y_{i,j}$;

$f$ being a transformation function of the data transformation model for representing the set of transformed variables in the latent data representation space;

$g$ being a decoding function of a decoder of the autoencoder; and

$||...||$ being a predetermined norm;

- step b) includes using a prediction model to determine the index value, the prediction model being trained based on a second training dataset including at least one set of reference transformed variables, each set of reference transformed variables being associated to a respective label indicating an evolution, over time, of a corresponding hearth-related condition of a respective second reference subject;

- the prediction model is a recursive neural network, preferably a long short-term memory;

- the method further comprises a step of joint training for jointly training the data space transformation model and the prediction model based on a global training dataset including at least one set of global reference inputs, each global reference input of a given set of global reference inputs being representative of an evolution, over time, of a respective health variable of a respective third reference subject, each set of global reference inputs being further associated to a label indicating whether the respective third reference subject has experienced an unfavorable outcome of the heart-related condition, the set of reference transformed variables input to the prediction model during the step of joint training being the set of transformed variables determined by the data space transformation model, during the step of joint training, based on each global reference inputs;

- a dimension of the latent data representation space is lower than a number of inputs in the set of inputs;

- the method further comprises, for each heart-related condition, outputting an alert signal, representative of an unacceptably high risk of experiencing an unfavorable outcome of the heart-related condition, if at least one corresponding index value is outside a range associated to the patient;

- each health variable is retrieved from at least one of: an implantable medical device of the patient, a health record of the patient or an excerpt of the health record of the patient, an insertable medical device of the patient and a smart device of the patient, such as a wearable smart device, an app, a dedicated database or a website, based on self-declared information provided by the patient;

- the unfavorable outcome of the at least one heart-related condition is one of: a worsening of a heart failure, an occurrence of myocardial infarction, an occurrence or a worsening of atrial fibrillation, an occurrence of a stroke, or an occurrence or a worsening of sleep apnea;

- the method further comprises, for each determined index value, outputting an information relating to n inputs that contribute the most to the determined index value, n being a predetermined integer.

The invention also relates to a computer program product comprising instructions which, when the program is executed by a computer, cause the computer to perform the method as previously defined.

The invention also relates to a prediction system prediction system for predicting an evolution of at least one heart-related condition of a patient, the prediction system including a processing unit (4) configured to, for each heart-related condition:

a) compute a set of transformed variables based on a set of inputs, at least part of the set of inputs being representative of a temporal evolution of a corresponding health variable among a predetermined set of health variables of the patient, the set of transformed variables being a representation of the set of inputs in a predetermined latent data representation space;

b) determine at least one index value based on the computed set of transformed variables, each index value being representative of a respective risk of an unfavorable outcome of the heart-related condition.

## BRIEF DESCRIPTION OF THE DRAWINGS

**Figure 1** is a schematic representation of a prediction system according to the invention; and

**Figure 2** is a flowchart of the method performed by the prediction system of figure 1.

## DETAILED DESCRIPTION

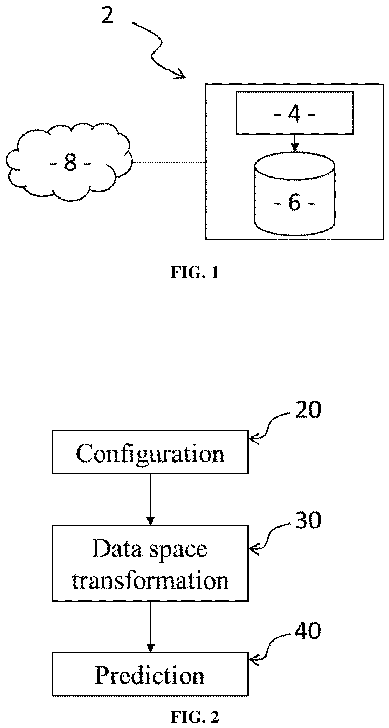

A prediction system 2 according to the invention is shown on figure 1.

### General description of the prediction system 2

The prediction system 2 is configured to predict an evolution of at least one heart-related condition of a patient.
More precisely, the prediction system 2 is configured to determine, for each heart-related condition, based at least on a temporal evolution of one or more predetermined variables of interest (including health variables) of the patient, at least one index value, each representative of a risk of an unfavorable outcome of said heart-related condition. Advantageously, the index value is representative of a risk of an unfavorable outcome of a given heart-related condition at a given time horizon.
Preferably, the unfavorable outcome of the at least one heart-related condition is one of: a worsening of a heart failure, an occurrence of a myocardial infarction, an occurrence or a worsening of atrial fibrillation, an occurrence of a stroke, or an occurrence or a worsening of sleep apnea. Indeed, sleep apnea is known to lead to cardiac disorders such as ventricular arrhythmias (risk is increased by 2 to 4 times).
As shown on figure 1, the prediction system comprises a processing unit 4. The prediction system 2 may also comprise an optional memory 6.
According to the invention, the expression "processing unit" should not be construed to be restricted to hardware capable of executing software, and refers in a general way to a processing device, which can, for example, include a microprocessor, an integrated circuit, or a programmable logic device (PLD). The processing unit 4 may also encompass one or more Graphics Processing Units (GPU) or Tensor Processing Units (TSU), whether exploited for computer graphics and image processing or other functions. Additionally, the instructions and/or data enabling to perform associated and/or resulting functionalities may be stored on any processor-readable medium such as, e.g., an integrated circuit, a hard disk, a CD (Compact Disc), an optical disc such as a DVD (Digital Versatile Disc), a RAM (Random-Access Memory) or a ROM (Read-Only Memory). Instructions may be notably stored in hardware, software, firmware or in any combination thereof.
The processing unit 4 is configured to determine, for each heart-related condition, the aforementioned index value.
More precisely, for each heart-related condition, the processing unit 4 is configured to receive a set of inputs relating to the patient. The set of inputs is received over a communication network 8, and/or is stored on the memory 6.
For each patient, the set of inputs is representative of an evolution, over time, of predetermined variables of interest associated to the said patient.
For instance, for each patient, at least part of the set of inputs is representative of a temporal evolution of a corresponding health variable among a predetermined set of health variables of said patient.
Another part of the set of inputs may be representative of medical, behavioral or social conditions, such as: genotype, phenotype, permanent atrial fibrillation, hypertension, diabetes, obesity, smoking, alcohol, sedentary lifestyle.
For instance, the health variables represented by the set of inputs include at least one of (not limited to the list): heart rate (at rest, at exercise, circadian variations...), activity level (accelerometry and/or minute ventilation), heart rate variability (such as time-domain or frequency-domain parameters, linear or non-linear parameters...), atrial and ventricular arrhythmias (such as atrial burden, trends, types, circadian variations...), pacing percentage, sensing percentage, P-wave and R-wave amplitudes, cardiac status (such as left ventricular ejection fraction or LVEF), biology parameters (such as BNP, NT-proBNP, TNF-$\alpha$, renal function...), signs (such as oedema, elevated jugular venous pressure, crepitation or wheeze, tachycardia, third heart sound...) and symptoms (such as dyspnea, reduced exercise tolerance, fatigue...). Some data specific to an implantable device can be used as a health variable, such as transthoracic impedance or heart sounds.
Preferably, each health variable is retrieved from at least one of: an implantable medical device of the patient, a health record of the patient or an excerpt of the health record of the patient, an insertable medical device of the patient and a smart device of the patient such as a wearable smart device, a smart balance, a tensiometer, and so on. Such health variable may also be retrieved from an app, a dedicated database or a website, based on self-declared information provided by the patient.

As an example, the excerpt of the health record preferably includes data such as: age, sex, medication taken by the patient, and data of the health record that are related to medical, social and behavioral parameters of the patient. This is relevant because it is well established that the course of heart-related conditions is related to age and gender, and can be modified by the drug regimen (such as anti-arrhythmic, anti-coagulant...). Comorbidities can have a deleterious impact of the cardiac condition such as diabetes and hypertension. Alcohol abuse and smoking are behaviors that can impact the course of the health-related conditions.

For each patient, referenced "i", the inputs of the corresponding set of inputs, hereinafter noted, $Y_{i,j}$, correspond to the acquired values of the variables of interest (including the health variables) at various successive instants (or "timestamps") $t_j$. In other words, at instant $t_j$, and for patient i, j successive vectors Yi have been acquired.

Since the actual values of all the variables of interest (noted $X_{i,j}$, and which include the health variables) are not available at all times, the set of inputs $Y_{i,j}$ can be regarded as the actual values $X_{i,j}$ of all the variables of interest, to which a mask $u_{i,j}$ has been applied. As an example, $Y_{i,j} = X_{i,j}u_{i,j}$. Said mask $u_{i,j}$ may vary from a timestamp to another.

The mask $u_{i,j}$ is therefore representative of potential missing values and data. For patient i and at timestamp $t_j$, $u_{i,j}$ is, for instance, a square matrix having zero as non-diagonal coefficients, and ones as diagonal coefficients, except for coefficients that are associated to values and data of vector $X_{i,j}$ that are not available; in this case, said diagonal coefficients also have a zero value.

Furthermore, the processing unit 4 is configured to compute a set of transformed variables (also called "projected variables") based on the set of inputs relating to the patient. More precisely, the set of projected variables is a representation of the set of inputs $Y_{i,j}$ in a predetermined latent data representation space. In other words, to each timestamp $t_j$ corresponds a respective value for each projected variable of the set of projected variables.

The set of transformed variables is noted $f(Y_{i,j})$, f being a predetermined data transformation function (also called "projection function"). As will be explained later, the dimension of $f(Y_{i,j})$ is not necessarily lower than the dimension of $Y_{i,j}$.

Preferably, each heart-related condition is associated to a corresponding latent data representation space.

Preferably, for each heart-related condition, the corresponding latent data representation space has a dimension that is lower than the number of inputs in the set of inputs. Such feature is advantageous, as it generally leads to noise reduction of the temporal variables of interest (amongst which the predetermined health variables) represented by the set of inputs.

Nevertheless, depending on the heart-related condition, a dimension of the latent data representation space that is higher than the number of inputs in the set of inputs may also be envisaged. This may be advantageous, as such feature may allow to better take into account constraints related to the implementation of the projection function.

The determination of the latent data representation space, as well as of the projection function f, is performed during configuration of the prediction system 2, as will be disclosed later.

Finally, for each heart-related condition, the processing unit 4 is configured to determine, based on the computed set of projected variables, the corresponding index value that is representative of the respective risk of an unfavorable outcome of the heart-related condition.

Preferably, the processing unit 4 is configured to output an alert signal, representative of an unacceptably high risk, for the patient, of experiencing an unfavorable outcome of the heart-related condition, if a corresponding index value is outside a range associated to the patient. Such range may be predetermined or auto-adaptive (for instance based on an evolution of the health status of the patient or the change of a constant status, e.g., onset of diabetes).

Preferably, the processing unit 4 is also configured to output, for each determined index value, an information relating to n inputs that contribute the most to the determined index value, n being a predetermined integer. For instance, a relationship between the inputs and the latent data representation space is determined (for instance using a decision tree correlating clusters in the latent data representation space and corresponding inputs of the prediction system 2), as well as a relationship between the latent data representation space and the index values (for instance, based on Shapley values, to link each index value to the transformed variables that have a positive contribution to the index value, or the most important contribution to the index value). In such way, for each index value, the transformed variables that have the highest contribution to said index value are identified, therefore allowing identification of the inputs that most contribute to said determined index value (thanks to the established relationship between the latent data representation space and the inputs). Such information may be helpful for the healthcare provider in providing appropriate recommendations to the patient, or taking appropriate action.

## Configuration of the prediction system 2

*Data space transformation model*

Preferably, in order to compute the projected variables, the processing unit 4 is configured to implement a data space transformation model.

In this case, the data space transformation model has been previously trained in order to determine the latent data representation space. More precisely, for each heart-related condition, the data space transformation model is trained based on a first training dataset including at least one set of reference inputs, each associated to a respective first reference subject. Even more precisely, for each set of reference inputs, each corresponding reference input is representative of an evolution, over time, of a respective variable of interest (such as a health variable) of the respective first reference subject.

Preferably, the first training dataset includes at least one healthy set of reference inputs. Each healthy set of reference inputs is associated to a healthy patient who has not experienced an unfavorable outcome of the heart-related condition. In this case, the data space transformation model is trained so that, in the latent data representation space, each projected variable of the set of reference projected variables corresponding to each healthy set of reference inputs has a predefined mean value $\mu$, for instance zero. This is advantageous, since the projected variables associated to a healthy patient will have values close to the predefined mean value $\mu$, while a patient at risk of experiencing an unfavorable outcome of the heart-related condition will be more likely to be associated to projected variables having values departing from said predefined mean value $\mu$, which will make detection of such patient easier.

Moreover, in this case, the data space transformation model is preferably also trained so that, in the latent data representation space, each projected variable of the set of reference projected variables corresponding to each healthy set of reference inputs is normally distributed. This feature is advantageous, as it leads to a better stability of the projection, especially if the data space transformation model includes an encoder, as will be described below.

This distribution is achieved by including, in the loss function during training of the data space transformation model, the following component:

$$\alpha \left\| f\left(Y_{i,j}\right) - \mu \right\|_2^2$$

$\alpha$ being a predetermined hyperparameter; and
$\|...\|_2$ being the Euclidean norm.

Preferably, to reinforce the independence of the data space transformation model with respect to the mask $u_{i,j}$, the loss function during training of the data space transformation model further includes the following component:

$$\left\| f\left(Y_{i,j}\right) - f\left(Y'_{i,j}\right) \right\|$$

$Y'_{i,j}$ being the result of multiplying the actual values $X_{i,j}$ by a random mask $u'_{i,j}$, preferably distinct from $u_{i,j}$; and
$\|...\|$ being a predetermined norm.

Since $X_{i,j}$ is generally not available, $Y'_{i,j}$ is obtained by multiplying $Y_{i,j}$ by a random mask $v_{i,j}$.

This component forces the projected variables to be the same regardless of the number and the nature of missing values, at a given time, for the inputs.

Preferably, to increase stability of the data space transformation model over changes of the mask $u_{i,j}$ from one timestamp to the next one, the loss function includes the following component:

$$\frac{\left| f\left(Y_{i,j}\right) - f\left(Y_{i,j+1}\right) \right|}{\left| Y_{i,j} - Y_{i,j+1} \right|^2}$$

Such component results from the fact that, over time, the values $X_{i,j}$ of the variables of interest (including the health variables) can be regarded as following a Markov chain, with potential value jumps and/or plateaus. Therefore, this component of the loss function constrains the projected variables and prevents strong variations originating from mere fluctuations of the set of inputs over time, unless the variations of Yi from one timestamp to the other exceed a certain amount.

For instance, the loss function during training of the data space transformation model includes a sum of the aforementioned components.

Advantageously, the data space transformation model is an autoencoder including an encoder. In this case, the encoder is designed to receive the set of inputs, the set of projected variables $f(Y_{i,j})$ being an output of the encoder, and the projection function f being an encoding function associated to the encoder.

Moreover, the autoencoder preferably includes a decoder, associated to a decoding function g, so that an output of the autoencoder, when provided with the set of inputs $Y_{i,j}$, is g of($Y_{i,j}$).

In this case, the loss function used for training the data space transformation model includes the following component:

$$\left\| Y_{i,j} - \left( g \circ f\left(Y_{i,j}\right)\right) u_{i,j} \right\|$$

This ensures that the projected variables are a good representation of the inputs in the latent data representation space.

In this case, the loss function used for training the data space transformation model preferably also includes the following component:

$$\left\| Y'_{i,j} - \left( g \circ f\left(Y'_{i,j}\right)\right) v_{i,j} \right\|$$

Moreover, by training the data space transformation model, the latent data representation space is obtained. The dimension of the latent data representation space may be a fixed parameter during the training of the data space transformation model.

Alternatively, computation of the projected variables does not involve implementing an artificial intelligence model. In this case, the processing unit 4 is, for instance, configured to perform a data projection algorithm, such as a data reduction algorithm (e.g., principal component analysis). In this case, the latent data representation space is obtained by performing the data projection algorithm on the first training dataset.

*Prediction model*

Advantageously, in order to determine each index value, the processing unit 4 is further configured to implement a prediction model.

In this case, the prediction model has been previously trained based on a second training dataset including at least one set of reference projected variables, each set of reference projected variables being associated to a respective label indicating an evolution, over time, of a corresponding hearth-related condition of a respective second reference subject. Said label may, for instance, have the values "has experienced an unfavorable outcome of the heart-related condition" or "has not experienced an unfavorable outcome of the heart-related condition". In the case where the unfavorable outcome is hospitalization, said label has, for instance, the values "has been hospitalized" or "has not been hospitalized".

The second training dataset is, for instance, the result of projecting the first training dataset in the previously determined latent data representation space.

Advantageously, in the case where the reference projected variables correspond to a subject who has been hospitalized, each reference projected variable is further associated to a duration between the corresponding timestamp $t_{i,j}$ and the date of the unfavorable outcome, such as hospitalization. This allows the prediction model, once trained, to compute, for each patient, index values that are each representative of a risk of an unfavorable outcome of a given heart-related condition at a given time horizon.

The prediction model is, advantageously, a recursive neural network, preferably a long short-term memory. Indeed, such neural networks are well suited for classification based on time series. Preferably, in this case, training of the long short-term memory involves a cross-entropy loss.

Using a long short-term memory is also advantageous because such neural network is configured to process data based on a short-term time frame and a long-term time frame, longer than the short-term time frame, thus mimicking the behavior of a healthcare professional during diagnosis.

Advantageously, configuration of the prediction system further comprises a step of joint training.

Such step of joint training is aimed at jointly training the data space transformation model and the prediction model based on a global training dataset including at least one set of global reference inputs.

In this case, each global reference input of a given set of global reference inputs is representative of an evolution, over time, of a respective variable of interest (such as a health variable) of a respective third reference subject. Moreover, in this case, each set of global reference inputs is further associated to a label indicating whether the respective third reference subject has experienced an unfavorable outcome of the heart-related condition.

During this step of joint training, the set of projected variables determined by the data space transformation model, based on each global reference inputs, forms the set of reference projected variables input to the prediction model.

Moreover, at the end of this step of joint training, the latent data representation space may differ from the latent data representation space obtained during training of the data space transformation model alone.

During such joint training, the loss function for the data space transformation model preferably includes the components detailed above.

Moreover, during such joint training, the loss function for the prediction model is, preferably, a cross-entropy loss.

For instance, the set of global reference inputs is the first training dataset, or includes part of the first training dataset.

## Operation of the prediction system

Operation of the prediction system 2 will now be disclosed.

During a configuration step 20, the prediction system 2 is configured, and the latent data representation space is determined. More precisely, in the case where the prediction system 2 is model-based, each of the corresponding data space transformation model and prediction model is trained.

Preferably, the data space transformation model and the prediction model are also jointly trained. In this case, the loss function is advantageously:

$$
\mathcal{L} = \left\| Y_{i,j} - \left( g \circ f(Y_{i,j}) \right) u_{i,j} \right\| + \left\| Y'_{i,j} - \left( g \circ f(Y'_{i,j}) \right) v_{i,j} \right\| + \alpha \left\| f(Y_{i,j}) - \mu \right\|_2^2 + \left\| f(Y_{i,j}) - f(Y'_{i,j}) \right\|
$$

$$
+ \frac{\left| f(Y_{i,j}) - f(Y_{i,j+1}) \right|}{\left| Y_{i,j} - Y_{i,j+1} \right|^2}
$$

Then, for a given patient, a set of inputs Yij, at least part of which is representative of a temporal evolution of a corresponding health variable among a predetermined set of health variables of the patient, is provided to the prediction system 2.

Then, during data space transformation step 30, the processing unit 4 computes the transformed variables $f(Y_{i,j})$, which represent the inputs $Y_{i,j}$ of the set of inputs in the latent data representation space. Preferably, step 30 is based on the implementation of the trained data space transformation model mentioned above.

Then, during prediction step 40, each index value corresponding to each heart-related condition is computed. Preferably, step 40 is based on the implementation of the trained prediction model mentioned above.

Preferably, the processing unit 4 outputs an alert signal, representative of an unacceptably high risk, for the patient, of experiencing an unfavorable outcome of the heart-related condition, if an index value is outside a given range associated to the patient.

## Claims

1. A computer-implemented method for predicting an evolution of at least one heart-related condition of a patient, the method including, for each heart-related condition:

   a) based on a set of inputs, at least part of the set of inputs being representative of a temporal evolution of a corresponding health variable among a predetermined set of health variables of the patient, computing a set of transformed variables, the set of transformed variables being a representation of the set of inputs in a predetermined latent data representation space;
   b) determining, based on the computed set of transformed variables, at least one index value, each representative of a respective risk of an unfavorable outcome of the heart-related condition.

2. The method according to claim **1,** wherein step a) includes using a data space transformation model to compute the set of transformed variables, the data space transformation model being trained in order to determine the latent data representation space based on a first training dataset including at least one set of reference inputs, each reference input of a given set of reference inputs being representative of an evolution, over time, of a respective health variable of a respective first reference subject.

3. The method according to claim **2,** wherein the first training dataset includes at least one healthy set of reference inputs associated to a corresponding healthy patient who has not experienced an unfavorable outcome of the heart-related condition, the data space transformation model being trained so that, in the latent data representation space, each transformed variable of the set of reference transformed variables corresponding to each healthy set of reference inputs has a predefined mean value and is, preferably, normally distributed.

4. The method according to claim **2,** wherein the loss function, during training of the data space transformation model, includes at least one of the following components:

$$\alpha \left\| f(Y_{i,j}) - \mu \right\|_2^2$$
$$\left\| f(Y_{i,j}) - f(Y'_{i,j}) \right\|$$
$$\frac{\left| f(Y_{i,j}) - f(Y_{i,j+1}) \right|}{\left| Y_{i,j} - Y_{i,j+1} \right|^2}$$

$Y_{i,j}$ being the inputs for patient i at timestamp $t_j$;
$Y'_{i,j}$ being inputs generated by applying a random mask $v_{i,j}$ to $Y_{i,j}$;
f being a transformation function of the data transformation model for representing the set of transformed variables in the latent data representation space;
$\alpha$ being a predetermined hyperparameter;
$\|...\|$ being a predetermined norm; and
$\|...\|_2$ being the Euclidean norm.

5. The method according to any one of claims **2** to **4,** wherein the data space transformation model is an autoencoder including an encoder for receiving the set of inputs, the set of transformed variables being an output of the encoder.

6. The method according to claim **5,** wherein the loss function, during training of the data space transformation model, includes at least one of the following components:

$$\left\| Y_{i,j} - \left( g \circ f(Y_{i,j}) \right) u_{i,j} \right\|$$
$$\left\| Y'_{i,j} - \left( g \circ f(Y'_{i,j}) \right) v_{i,j} \right\|$$

$Y_{i,j}$ being the inputs for patient i at timestamp $t_j$;
$Y'_{i,j}$ being inputs generated by applying a random mask $v_{i,j}$ to $Y_{i,j}$;
f being a transformation function of the data transformation model for representing the set of transformed variables in the latent data representation space;
g being a decoding function of a decoder of the autoencoder; and
$\|...\|$ being a predetermined norm.

7. The method according to any one of claims **1** to **6,** wherein step b) includes using a prediction model to determine the index value, the prediction model being trained based on a second training dataset including at least one set of reference transformed variables, each set of reference transformed variables being associated to a respective label indicating an evolution, over time, of a corresponding hearth-related condition of a respective second reference subject.

8. The method according to claim **7,** wherein the prediction model is a recursive neural network, preferably a long short-term memory.

9. The method according to claim **7** or **8** when dependent on any one of claims **2** to **6,** further comprising a step of joint training for jointly training the data space transformation model and the prediction model based on a global training dataset including at least one set of global reference inputs,

each global reference input of a given set of global reference inputs being representative of an evolution, over time, of a respective health variable of a respective third reference subject,
each set of global reference inputs being further associated to a label indicating whether the respective third reference subject has experienced an unfavorable outcome of the heart-related condition,
the set of reference transformed variables input to the prediction model during the step of joint training being the set of transformed variables determined by the data space transformation model, during the step of joint training, based on each global reference inputs.

10. The method according to any one of claims **1** to **9,** wherein a dimension of the latent data representation space is lower than a number of inputs in the set of inputs.

11. The method according to any one of claims **1** to **10,** further comprising, for each heart-related condition, outputting an alert signal, representative of an unacceptably high risk of experiencing an unfavorable outcome of the heart-related condition, if at least one corresponding index value is outside a range associated to the patient.

12. The method according to any one of claims **1** to **11,** wherein each health variable is retrieved from at least one of: an implantable medical device of the patient, a health record of the patient or an excerpt of the health record of the patient, an insertable medical device of the patient and a smart device of the patient, such as a wearable smart device, an app, a dedicated database or a website, based on self-declared information provided by the patient.

13. The method according to any one of claims **1** to **12,** wherein the unfavorable outcome of the at least one heart-related condition is one of: a worsening of a heart failure, an occurrence of myocardial infarction, an occurrence or a worsening of atrial fibrillation, an occurrence of a stroke, or an occurrence or a worsening of sleep apnea.

14. The method according to any one of claims **1** to **13,** further comprising, for each determined index value, outputting an information relating to n inputs that contribute the most to the determined index value, n being a predetermined integer.

15. A computer program product comprising instructions which, when the program is executed by a computer, cause the computer to perform the method according to any one of claims **1** to **14.**

16. A prediction system for predicting an evolution of at least one heart-related condition of a patient, the prediction system including a processing unit (4) configured to, for each heart-related condition:

a) compute a set of transformed variables based on a set of inputs, at least part of the set of inputs being representative of a temporal evolution of a corresponding health variable among a predetermined set of health variables of the patient, the set of transformed variables being a representation of the set of inputs in a predetermined latent data representation space;
b) determine at least one index value based on the computed set of transformed variables, each index value being representative of a respective risk of an unfavorable outcome of the heart-related condition.

2

**FIG. 1**

20

Configuration

30

Data space
transformation

40

Prediction

**FIG. 2**

Europäisches
Patentamt

European
Patent Office

Office européen
des brevets

# EUROPEAN SEARCH REPORT

Application Number

EP 21 30 6385

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 111 830 A1 (KAUNAS UNIV OF TECH [LT]) 4 January 2017 (2017-01-04)<br>* paragraph [0011] *<br>* paragraph [0003] – paragraph [0004] *<br>* paragraph [0012] *<br>* paragraph [0017] *<br>* paragraph [0036] *<br>* paragraph [0037] * | 1–16 | INV.<br>G16H50/30 |
| X | US 2015/126883 A1 (AN QI [US] ET AL) 7 May 2015 (2015-05-07)<br>* paragraph [0027] *<br>* paragraph [0009] – paragraph [0010] *<br>* paragraph [0051] – paragraph [0052] *<br>* paragraph [0050] *<br>* paragraph [0055] – paragraph [0056] *<br>* paragraph [0058] – paragraph [0059] *<br>* paragraph [0066] *<br>* paragraph [0067] *<br>* paragraph [0075] – paragraph [0076] *<br>* paragraph [0078] *<br>* paragraph [0077] * | 1–16 | |

TECHNICAL FIELDS SEARCHED (IPC)

G16H

The present search report has been drawn up for all claims

| Place of search | Date of completion of the search | Examiner |
|---|---|---|
| Munich | 18 March 2022 | Megalou-Nash, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 30 6385

This annex lists the patent family members relating to the patent documents cited in the above-mentioned European search report.
The members are as contained in the European Patent Office EDP file on
The European Patent Office is in no way liable for these particulars which are merely given for the purpose of information.

18-03-2022

| Patent document cited in search report | | Publication date | Patent family member(s) | | Publication date |
|---|---|---|---|---|---|
| EP 3111830 | A1 | 04-01-2017 | CA | 2991001 A1 | 05-01-2017 |
| | | | EP | 3111830 A1 | 04-01-2017 |
| | | | US | 2016374580 A1 | 29-12-2016 |
| | | | WO | 2017001894 A1 | 05-01-2017 |
| US 2015126883 | A1 | 07-05-2015 | NONE | | |

EPO FORM P0459

For more details about this annex : see Official Journal of the European Patent Office, No. 12/82

**EP 4 160 617 A1**

**Patent documents cited in the description**

- US 9622664 B